# EUROPEAN PATENT SPECIFICATION

(11) **EP 2 231 613 B1**
(45) Date of publication and mention of the grant of the patent: **12.10.2011**
(21) Application number: 09719393.2
(22) Date of filing: 05.01.2009
(51) Int. Cl.: C07D 215/18, C07D 295/02, C07D 295/06

(54) **MONTELUKAST BENZHYDRYL PIPERAZINE SALTS AND PROCESS FOR PREPARATION THEREOF**
MONTELUKAST-BENZHYDRYL-PIPERAZIN-SALZE UND VERFAHREN ZU IHRER HERSTELLUNG
SELS DE PIPÉRAZINE DE BENZHYDRYLE DE MONTÉLUKAST ET LEUR PROCÉDÉ DE PRÉPARATION

(30) Priority: 07.01.2008 IN MU00382008
(43) Date of publication of application: 29.09.2010
(73) Proprietor: Torrent Pharmaceuticals Ltd., Gujarat (IN)
(72) Inventor: SHIMPI, Nitin, Ashok, Gujarat (IN); LAKKAD, Mahendra, Gordhanbhai, Gujarat (IN); NADKARNI, Sunil, Sadanand, Gujarat (IN); A.V.V., Srinivasa, Rao, Gujarat (IN)
(74) Representative: Srinivasan, Ravi Chandran
(86) International application number: PCT/IN2009/000011
(87) International publication number: WO 2009/113087

(56) References cited:
- WO-A-2007/096875

## Description

### FIELD OF THE INVENTION

The present invention relates to novel Benzhydryl piperazine salts of [R-(E)]-1-[[[1-[3-[2-(7-chloro-2-quinolinyl) ethenyl]phenyl]-3-[2-(1-hydroxy-1-methylethyl) phenyl] propyl] thio] methyl] cyclopropaneacetic acid represented by the formula (III). Furthermore, the invention relates to the use of novel Benzhydryl piperazine salts of [R-(E)]-1-[[[1-[3-[2-(7-chloro-2-quinolinyl) ethenyl]phenyl]-3-[2-(1-hydroxy-1-methylethyl) phenyl] propyl] thio] methyl] cyclopropaneacetic acid represented by the formula (III) for the preparation of substantially pure [R-(E)]-1-[[[1-[3-[2-(7-chloro-2-quinolinyl) ethenyl]phenyl]-3-[2-(1-hydroxy-1-methylethyl) phenyl] propyl] thio] methyl] cyclopropane acetic acid or its alkali salts and pharmaceutical composition comprising the same.

### BACKGROUND OF THE INVENTION

[R-(E)]-1-[[[1-[3-[2-(7-chloro-2-quinolinyl)ethenyl]phenyl]-3-[2-(1-hydroxy-1-methylethyl) phenyl] propyl] thio] methyl] cyclopropaneacetic acid sodium salt, also known by the name montelukast sodium, is represented by the structural formula I below:

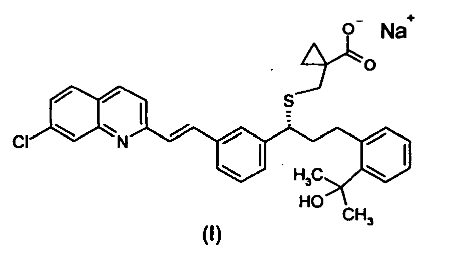

Montelukast is a leukotriene D4 antagonist. Montelukast is indicated for the prophylaxis and chronic treatment of asthma in adults and pediatric patients. It is also indicated for the relief of symptoms of seasonal allergic rhinitis and for perennial allergic rhinitis in adults and pediatric patients.

Montelukast sodium is a hygroscopic, optically active and white to off-white powder, which is freely soluble in methanol, ethanol, and water and practically insoluble in acetonitrile. Moreover, it is highly susceptible to degradation.

Montelukast sodium salt is available in a number of oral formulations including tablets, chewable tablets and oral granules. Montelukast sodium is marketed in the USA and other countries by Merck & Co., Inc. under the trade name Singulair ®.

Montelukast sodium and related compounds were first disclosed in European Patent No. EP480717. EP480717 discloses certain substituted quinoline compounds including [R-(E)-1-[[[1-[3-[2-[7-chloro-2-quinolinyl]ethenyl] phenyl]-3-[2-(1-hydroxy-1-methylethyl)phenyl] propyl]thio]methyl]cyclopropaneacetic acid sodium salt (Montelukast sodium), method for their preparation, pharmaceutical formulation using these compound and method of treatment of mammals especially humans.

EP 480717 discloses a process for preparing montelukast sodium by condensing 2-(2-(2-(3 (S)-(3-(2-(7-chloro-2-quinolinyl)-ethenyl)phenyl)-3-(methanesulfonyloxy)propyl) phenyl)-2-propoxy)tetra hydro pyran with Methyl 1-(acetylthiomethyl)cyclopropane acetate in presence of hydrazine, cesium carbonate in acetonitrile as solvent to get methyl ester of Montelukast in tetrahydro pyran protected form, which is further reacted with pyridinium p-toluene sulfonate, sodium hydroxide in a mixture of methanol and tetrahydrofuran as a solvent to afford Montelukast sodium of Formula (I), which is depicted in Scheme-1. In the mentioned process chromatographic techniques are used for purification of the methyl ester intermediate, which limits industrial usability of the method. Moreover, montelukast sodium, obtained by this method, is the oily substance. Many efforts have been made to purify and stabilize the sodium salt which is depicted herein below.

US 5,614,632, discloses a method of preparing crystalline montelukast sodium, which involves the preparation of the dilithium dianion of 1- (mercaptomethyl)cyclopropaneacetic acid, followed by condensation thereof with 2- (2-(3 -(S)-(3 -(7-chloro-2-quinolinyl)ethenyl)phenyl)-3 -methanesulfonyloxypropyl)- phenyl)-2-propanol, to yield montelukast acid as a viscous oil. It is further converted to its corresponding sodium salt via dicyclohexyl amine salt.

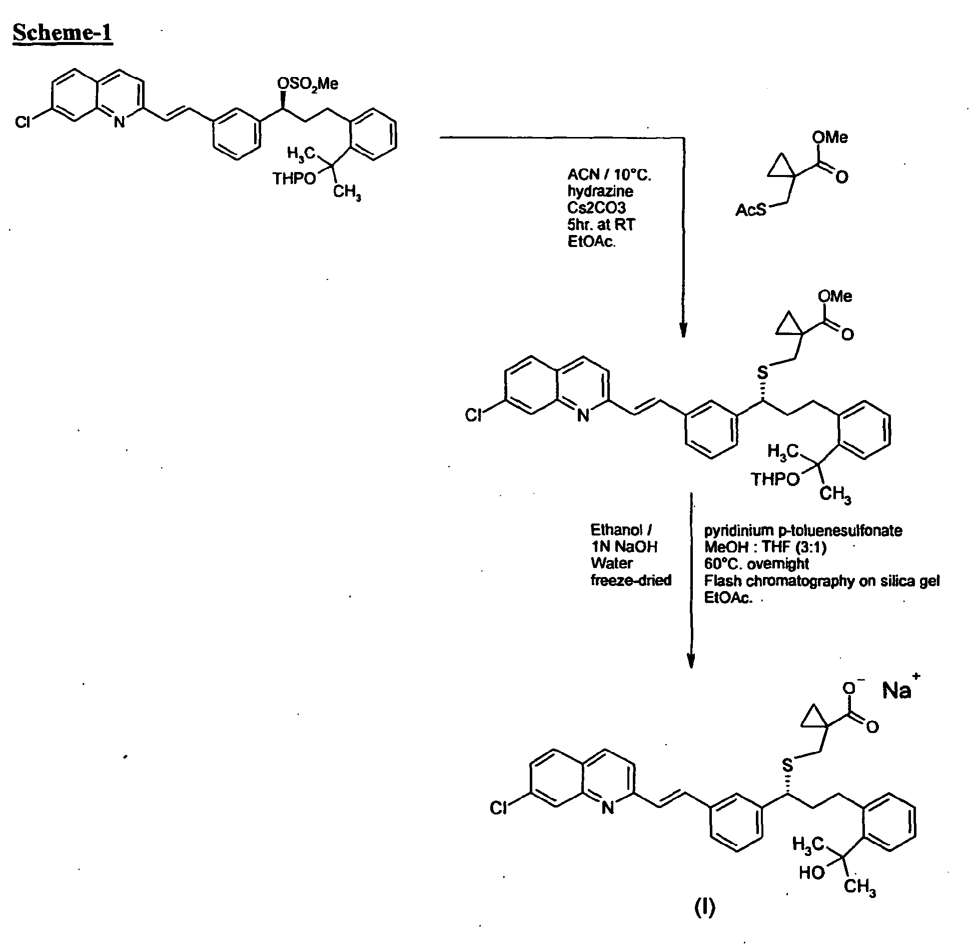

The above prior art i.e. EP 480717 & US 5,614,632 involve more number of steps, which include a series of protections and deprotection of 2-propanol i.e. diol intermediate, usage of unsafe raw materials such as n-butyl lithium, hydrazine, pyridinium p-toluenesulfonate and end-up with tedious workup to isolate the required product and thus results in excess time cycle, which in turn rendering the process more costly and less eco friendly. Thus, the process is not feasible for commercial scale up.

In US patent application no. 20050234241, Montelukast is prepared by mesylation of 2-(2-(2-(3(S)-(3-(2-(7-chloro-2-quinolinyl) ethenyl) phenyl)-3-(methanesulfonyloxy) propyl) phenyl)-2-propanol using methane sulfonyl chloride and condensation of resulting mesylated with cyclopropyl derivative. This compound is then hydrolyzed to afford montelukast, and it's isolation into amine salt of montelukast. The amine salt of montelukast is then converted into sodium salt of Montelukast.

US 2005/0107612 describes a process for preparing the t- butyl and phenethyl ammonium salts of montelukast acid in the purification process, as depicted in scheme-2. The mesylated derivative is first converted to the dicyclohexyl ammonium salt , which undergoes Grignard reaction and the resultant product is converted into the tert-butyl ammonium salt or to the phenethyl ammonium salt of montelukast acid. The salt is finally converted to the corresponding montelukast sodium salt. This process also posses several drawbacks like; two different amine salt preparations at two different stages, Grignard reaction in the last stage may affect the stereochemistry of the compound as well as generate several impurities, which are difficult to remove.

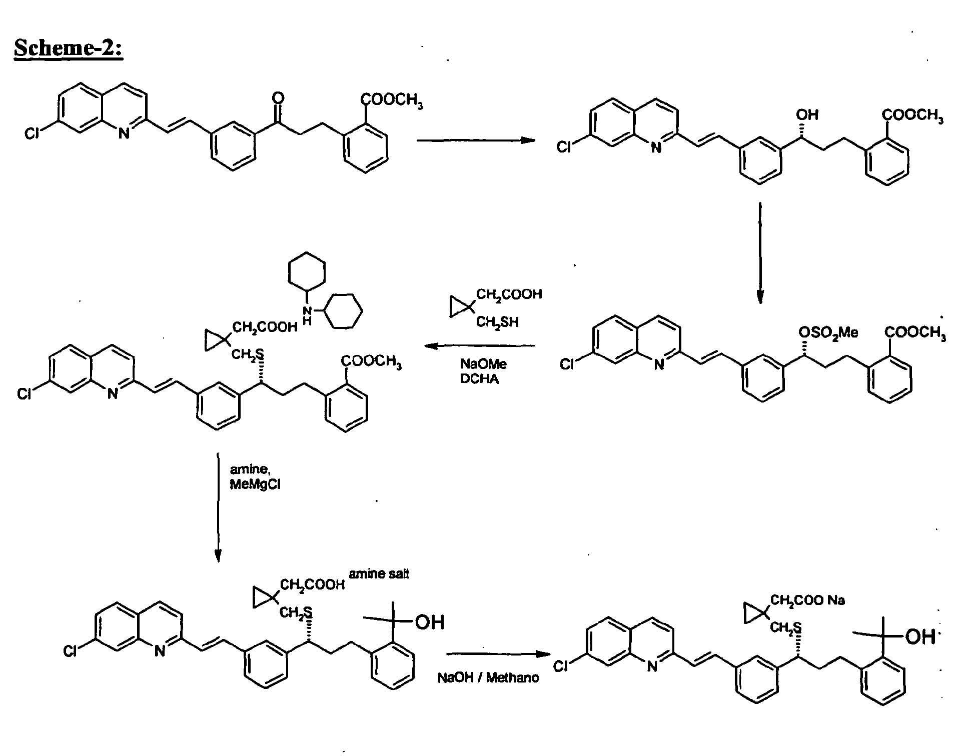

The use of the tert-butyl ammonium salt of montelukast acid in the preparation of montelukast sodium is disclosed in WO 2006/043846.

An international application WO 2006/008751 discloses the use of 3- halopropyl derivative for the preparation of Montelukast and its salts.

An international application WO 2007/004237 discloses α-methylbenzyl, dicyclohexyl, and cyclohexylethyl ammonium salts of Montelukast for preparing montelukast sodium,. Both prior arts i.e. WO 2006/008751 & WO 2007/004237 require twice amine salt preparations at two different stages for the preparation of Montelukast sodium, which renders process lengthy and cumbersome.

An international application WO 2007/005965 recites using the dipropyl ammonium salt of montelukast acid for preparing purified montelukast sodium.

An application WO 2007/096875 discloses the cycloalkyl amine salts of Montelukast acid such as cyclopentyl amine, cyclohexyl amine, cycloheptyl amine, cyclodocecyl amine, cyclooctyl amine and phenylethyl amine salt.

An application WO 2007/107297 is disclosing amantadine salt of Montelukast for preparing montelukast sodium. This prior art discloses that the known organic amines that have been used for this same purpose, i.e. the DCHA or TBA, are flammable, toxic and irritant liquids which are unstable in open air and of unpleasant smell. For instance, TBA boils already at about 45° C and DCHA is also a high boiling flammable liquid. Thus, in using them during chemical synthesis, special precautions may be necessary to protect the worker and the environment.

The purity of montelukast acid is affected by various factors like purity of the starting material, as well as conditions of mesylation, wherein increased reaction temperature results in decreased selectivity of mesylation of the secondary hydroxyl group, the type of the reaction solvent has an impact on reactivity of the diol-intermediate, an intramolecular substitution resulting in formation of a cyclic ether is observed in acidic medium. Like any synthetic compound, Montelukast can also contain process impurities, including unreacted starting materials, chemical derivatives of impurities contained in starting materials, synthetic by-products, and degradation products. Impurities in API are undesirable and some time their presence in excessive amount may be harmful to health of the patient. Hence the purity of the API produced in the commercial manufacturing process is a necessary condition for commercialization.

There are many patent applications which have tried to isolate montelukast free acid in solid form in an effort to purify and stabilize montelukast sodium salt.

Particularly, when API is desired to obtain in amorphous form, in particular case it may happen that even after the number of purification at amorphous stage might not provide the optimum purity of API. Furthermore, crystallization or purification at the final stage might change the nature of solid state which will not be required, when specific form of the compound is desirable to obtain specific dissolution. The same was observed during experimentation when Montelukast sodium was obtained into amorphous state directly from the Montelukast acid without any intermediate stage of salt preparation and crystallization of Montelukast acid. It was found difficult to remove the unrequired impurities even after number of purification without changing the nature of the compound.

As revealed above, it is apparent that till date, to obtain stable Montelukast sodium salt either montelukast acid is first converted to a different salt or as such montelukast acid is isolated in solid form to finally obtain a pure montelukast sodium salt in particularly in amorphous form. Further, US200701841 01, and US20070184108 reveal that composition prepared from the montelukast and its alkali salts undergoes degradation and thus it provides method of stabilizing montelukast in pharmaceutical composition. Thus, it can be said that there still exist a need to provide a stable montelukast and its composition.

Hence, it would be desirable to find a new pharmaceutically acceptable salt of Montelukast acid to facilitate the isolation and purification, as the known amine salts of montelukast suffer from multiple disadvantages.

Furthermore, there still exists a need to simplify the manufacturing process for preparation of montelukast acid and its alkali salts. Preparing novel salt of montelukast acid of greater degree of chemical purity is of special interest.

It was the aim of the present invention to develop novel salts of Montelukast having high purity with better yield, which is more viable for industrial use and its conversion to substantially pure montelukast and its alkali salts possessing advantageous physico-chemical properties, thermodynamically stable and with good reproducibility and therefore having appropriate processing parameters facilitating its formulating into the pharmaceutical dosage forms.

The aim of the invention has been achieved by obtaining novel Benzhydryl piperazine salts of Montelukast represented by the formula (III).

### SUMMARY OF THE INVENTION

The first embodiment of the present invention is to provide novel Benzhydryl piperazine salts of Montelukast represented by the formula (III). 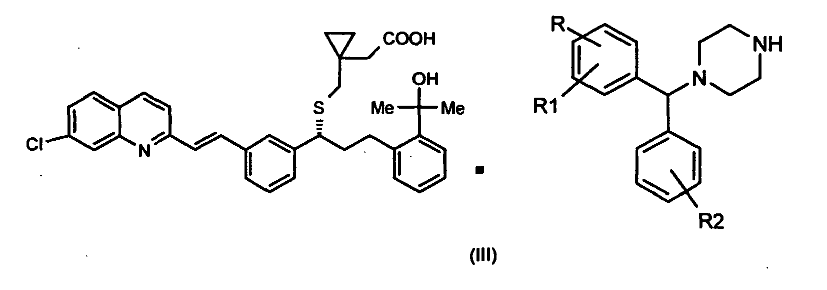 wherein;
R, R1, R2 is independently selected from hydrogen, halogen like F, Cl, Br &.I, (C1-C6) alkyl, (C1-C6) alkoxy, CH2F, CHF2 and CF3.

Another embodiment of the present invention is to provide novel Benzhydryl piperazine salts of Montelukast represented by the formula (III), which includes but not limited to Benzhydryl piperazine, 4-chloro-benzhydryl piperazine, 4-methoxy-benzhydryl piperazine, 3,5-dichloro-benzhydryl piperazine, 3,4-dichloro-benzhydryl piperazine, 4-fluoro-benzhydryl piperazine, 4-methyl-benzhydryl piperazine or 4-trifluoromethyl-benzhydryl-piperazine.

Yet another embodiment of the present invention is to provide Benzhydryl piperazine salts of Montelukast represented by the formula (III) having purity higher than 98%.

Yet another embodiment of the present invention is to provide Benzhydryl piperazine salt of Montelukast represented by the formula (III-a) having purity higher than 98%.

Yet another embodiment of the present invention is to provide 4-chloro-benzhydryl piperazine salt of Montelukast represented by the formula (III-b) having purity higher than 98%.

Yet another embodiment of the present invention is to provide Benzhydryl piperazine salts of Montelukast represented by the formula (III-c to III-h) having purity higher than 98%.

Yet another embodiment of the present invention is to provide the benzhydryl piperazines salts of Montelukast represented by the formula (III) in solid state, especially in crystalline state or in amorphous state.

Yet another embodiment of the present invention is to provide process for the preparation of benzhydryl piperazine salts of Montelukast represented by the formula (III) in solid state.

Yet another embodiment of the present invention is to provide the use of benzhydryl piperazine salts of Montelukast represented by the formula (III) for the preparation of substantially pure Montelukast or its alkali salts.

Yet another embodiment of the present invention is to provide the use of benzhydryl piperazine salts of Montelukast represented by the formula (III) for the preparation of substantially pure Montelukast or its alkali salts, which is crystalline or amorphous.

In one of the preferred embodiment, the present invention provides the use of benzhydryl piperazine & 4-chloro-benzhydryl piperazine salt of Montelukast for the preparation of Montelukast or its alkali salts having purity higher than 99%.

In another preferred embodiment, the present invention provides the use of benzhydryl piperazine salts of Montelukast represented by the formula (III) for the preparation of amorphous Montelukast sodium having purity higher than 99%.

In general embodiment present invention provides process for the preparation of Benzhydryl piperazine salts of Montelukast represented by the formula (III) from Montelukast of formula (II) and its conversion in to substantially pure montelukast or its alkali salts as described herein below:
(a) forming a solution of Montelukast of formula (II) in suitable solvent,
(b) adding benzhydryl piperazine derivative of formula (IV) to said solution obtained in step (a), 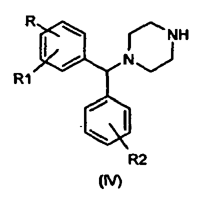 wherein;
   R, R1, R2 is independently selected from hydrogen, halogen like F, Cl, Br & I, (C1-C6) alkyl, (C1-C6) alkoxy, CH2F, CHF2 and CF3.
(c) isolating Benzhydryl piperazine salt of montelukast represented by the formula (III),
(d) optionally recrystallizing the benzhydryl piperazine salt of Montelukast represented by the formula (III) obtained in step (c),
(e) converting the said benzhydryl piperazine salt of Montelukast represented by the formula (III) into substantially pure Montelukast or its alkali salts.

The above reaction steps are schematically depicted in scheme-3 herein below.

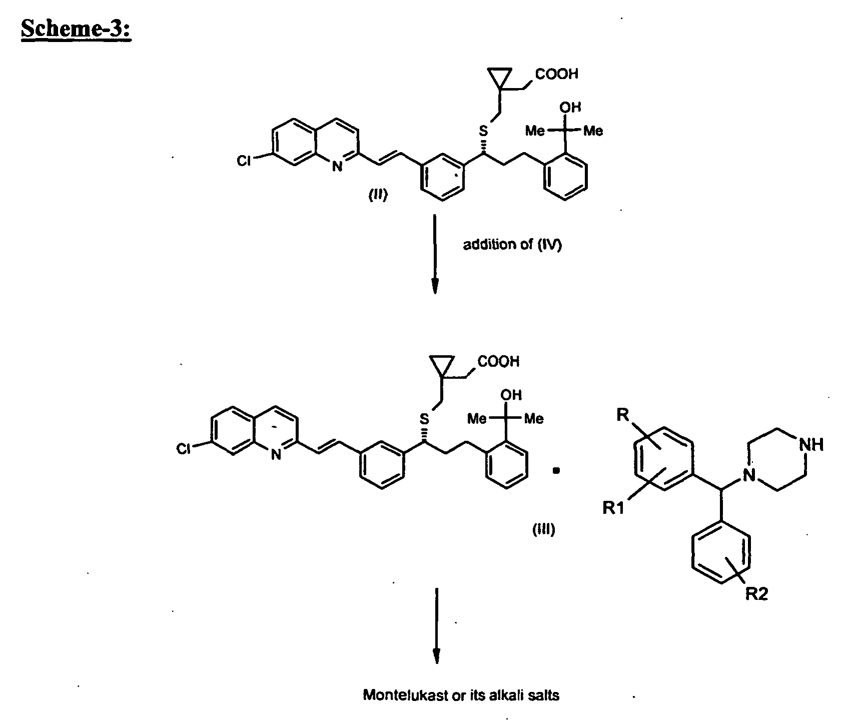

Wherein R, R1, R2 is independently selected from hydrogen, halogen like F, Cl, Br & I, Cl-C6 alkyl, (C1-C6) alkoxy, CH2F, CHF2 and CF3.

Yet another embodiment of the present invention is to provide the pharmaceutical composition of Benzhydryl piperazine salts of Montelukast represented by the formula (III).

Yet further embodiment of the present invention is to provide the pharmaceutical composition of Montelukast or its alkali salts prepared from the benzhydryl piperazine salts of Montelukast represented by the formula (III).

### BRIEF DESCRIPTION OF THE ACCOMPANYING DRAWINGS:

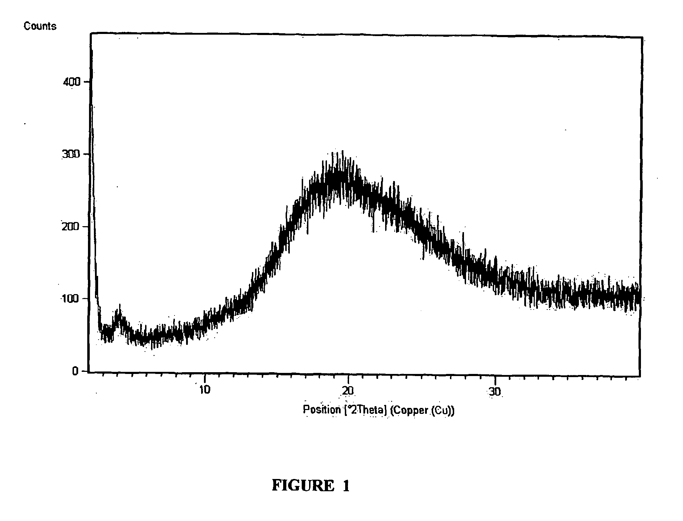

**Fig. 1**: This figure indicates X-ray diffraction pattern of amorphous Montelukast sodium.

### DETAILED DESCRIPTION OF THE INVENTION

The use of the terms "a" and "an" and "the" and similar referents in the context of describing the invention are to be construed to cover both the singular and the plural, unless otherwise indicated herein or clearly contradicted by context.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

For purposes of the present invention, the following terms are defined below.

The term "Montelukast" refers to [R-(E)]-1-[[[1-[3-[2-(7-chloro-2-quinolinyl) ethenyl] phenyl]-3-[2-(1-hydroxy-1-methylethyl) phenyl] propyl] thio] methyl] cyclopropaneacetic acid.

The term "Benzhydryl piperazine derivative" refers to the compound selected from the group comprising of Benzhydryl piperazine, 4-chloro-benzhydryl piperazine, 4-methoxy-benzhydryl piperazine, 3,5-dichloro-benzhydryl piperazine, 3,4-dichloro-benzhydryl piperazine, 4-fluoro-benzhydryl piperazine, 4-methyl-benzhydryl piperazine and 4-trifluoromethyl-benzhydryl-piperazineBenzhydryl piperazine or compound having benzhydryl piprazine moiety.

The term "substantially pure montelukast or its alkali salt" refers to montelukast or its alkali salt having purity higher than 99%.

As used herein, "Benzhydryl piperazine salt of montelukast" means any combination of benzhydryl piperazine derivative and montelukast, whether in solid state such as a crystalline substance or dissolved in a solvent. It also includes all polymorphic and pseudo- polymorphic salts, if any, including hydrates, etc. Generally Benzhydryl piperazine salts of montelukast can be represented by the formula (III) in the following way.

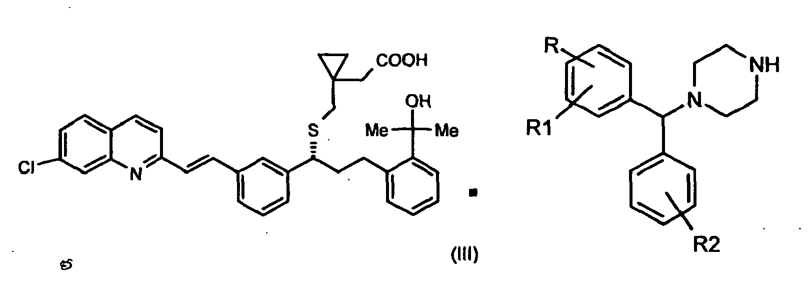

Wherein R, R1, R2 is independently selected from hydrogen, halogen like F, Cl, Br & I, C1-C6 alkyl, (C1-C6) alkoxy, CH2F, CHF2 and CF3.

The Benzhydryl piperazine salt of Montelukast represented by the formula (III) according to the present invention, which is selected from the group consisting of:
(a) Montelukast Benzhydryl Piperazine (III-a), wherein R = H, R1= H, R2 = H.
(b) Montelukast 4-Chloro-benzhydryl piperazine (III-b), wherein R= 4-Cl, R1 = H, R2 = H.
(c) Montelukast 4-methoxy-benzhydryl piperazine (III-c), wherein R=4-OMe, R1= H, R2=H.
(d) Montelukast 3,5-dichloro-benzhydryl piperazine (III-d), wherein R=3-Cl, R1=5-Cl, R2=H.
(e) Montelukast 3,4-dichloro-benzhydryl piperazine (III-e), wherein R=3-Cl, R1=4-Cl, R2=H
(f) Montelukast 4-fluoro-benzhydryl piperazine (III-f), wherein R=4-F, R1=H, R2=H
(g) Montelukast 4-methyl-benzhydryl piperazine (III-g), wherein. R=4-Me, R1=H, R2=H
(h) Montelukast 4-trifluoromethyl-benzhydryl-piperazine (III-h), wherein R=4-CF3, R1=H, R2=H

In general embodiment present invention provides process for the preparation of Benzhydryl piperazine salts of Montelukast represented by the formula (III) from Montelukast of formula (II) and its conversion in to substantially pure montelukast or its alkali salts as described herein below:
(a) forming a solution of Montelukast of formula (II) in suitable solvent,
(b) adding benzhydryl piperazine derivative of formula (IV) to said solution obtained in step (a), 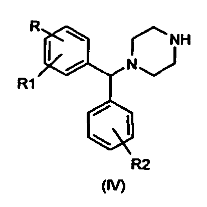 wherein;
   R, R1, R2 is independently selected from hydrogen, halogen like F, Cl, Br, I, (C1-C6) alkyl, (C1-C6) alkoxy, CH2F, CHF2 and CF3.
(c) isolating Benzhydryl piperazine salt of montelukast represented by the formula (III),
(d) optionally recrystallizing the benzhydryl piperazine salt of Montelukast represented by the formula (III) obtained in step (c),
(e) converting the said benzhydryl piperazine salt of Montelukast represented by the formula (III) into substantially pure Montelukast or its alkali salts.

Step a, b, c & d, Benzhydryl piperazine salt of Montelukast represented by the formula (III) can be prepared by forming solution of montelukast acid of formula (II) with Benzhydryl piperazine derivative of formula (IV) in suitable solvent at a temperature of 20-50° C for sufficient time, it may be 15-minute to 10 hours. The Benzhydryl piperazine salt of montelukast represented by the formula (III) can be isolated by using conventional techniques, like adding antisolvent, seeding of Benzhydryl piperazine salt of Montelukast represented by the formula (III), stirring the solution till the precipitate comes out. The obtained Benzhydryl piperazine salt of Montelukast represented by the formula (III) can be optionally re-crystallized by using one or more suitable solvent at ambient temperature. The obtained Benzhydryl piperazine salt of Montelukast represented by the formula (III) is further dried under vacuum for 2-8 hrs to obtain dry product at 35-65°C.

The suitable solvent used in formation of solution, isolation and crystallization of Benzhydryl piperazine salt of Montelukast represented by the formula (III) include but not limited to alcohols (methanol, ethanol, isopropanol, butanol, etc.), ketones (acetone, methyl isopropyl ketone, etc.), aliphatic ethers (diethyl ether, di tert. butyl ether, etc.), cyclic ethers ( tetrahydrofuran, dioxane, etc.), aliphatic esters (ethyl acetate, etc.), hydrocarbons (toluene, heptane, hexane, etc,), chlorinated solvent (chloroform, dichloromethane, etc.), acetonitrile, polar aprotic solvents (DMF, DMAc, etc.) or mixtures thereof.

The preferred solvent for forming solution of Montelukast acid of formula (II) with Benzhydryl piperazine derivatives of formula (IV) is aliphatic ester or acetone or chlorinated solvent or alcohol or hydrocarbons.

The preferred antisolvent used to isolate the Benzhydryl piperazine salts of Montelukast represented by the formula (III) is aliphatic ester, hydrocarbons or acetone; more preferred is heptane or acetone.

The preferred solvent used for the recrystallization of Benzhydryl piperazine salts of Montelukast represented by the formula (III) can be esters, acetone, hydrocarbon, alcohol or mixture thereof.

In preferred embodiment, the salt formation reaction by using Benzhydryl piperazine derivative of formula (IV) is useful for the purification of a crude montelukast acid, i.e. a residual Montelukast acid of formula (II) having 80% or less content of the Montelukast acid compound for the preparation of Montelukast sodium of formula (I). However such an impure starting material is used in the instant invention for the preparation of Benzhydryl piperazine salt of Montelukast represented by the formula (III), which, after isolation, has a far lower content of side products because Benzhydryl piperazine derivative of formula (IV) selectively form the salt preferably with Montelukast acid over its degradation product and process impurities; therefore they remain in the mother liquor and are removed/reduced in the desired product. A simple single treatment of a less pure Montelukast acid with Benzhydryl piperazine derivatives of formula (IV) provides salt having 98% and higher purity.

Another embodiment of present invention is the salt formation by using Benzhydryl piperazine derivative of formula (IV) with other chemically active compounds; wherein - COOH group is actively participate in salt formation e.g. statins like atorvastatin, simvastatin, rosuvastatin, sartans like valsartan; candesartan, telmisartan or etc.

Step e, Then, Montelukast Benzhydryl piperazine salt represented by the formula (III) may be either converted to the montelukast acid of formula (II), which can be isolated and then further converted into montelukast alkali salt or Montelukast Benzhydryl piperazine represented by the formula (III) may be converted into Montelukast alkali salt directly without in situ generating montelukast free acid of formula (II).

In one way, montelukast Benzhydryl piperazine salt represented by the formula (III) is combined with one or more solvent selected from the group consisting of water, water immiscible organic solvent such as aromatic hydrocarbon (benzene or toluene), aliphatic hydrocarbon (hexane or heptane), an aliphatic ester (ethyl acetate, etc.) or chlorinated hydrocarbon (chloroform, dichloromethane) at 20-40 °C. To this suspension, a water soluble organic acid is added, preferably acetic acid or hydrochloric acid. Then the reaction mixture is stirred for sufficient time at 20-40°C and the organic layer comprising the montelukast acid is then separated. The organic layer can be washed with water and dried on sodium sulfate and montelukast acid may be obtained in residual form by removal of the solvent by evaporation or by other known means.

Then obtained Montelukast acid represented by the formula (II) is treated with a source of alkali metal, for instance alkali salts used in step (e) may be selected from sodium hydroxide, sodium methoxide, sodium ethoxide, calcium hydroxide, calcium methoxide, calcium ethoxide, potassium hydroxide, potassium methoxide, potassium ethoxide, magnesium hydroxide, magnesium methoxide, magnesium ethoxide, etc. preferably the alkali is sodium hydroxide, in suitable solvent system, preferably in an alcoholic solvent such as methanol, ethanol, propanol, butanol, 2-propanol or tertiary butanol.

Then the Montelukast alkali salt can be isolated in crystalline from or amorphous form by technique known in prior art.

In one embodiment the solution of montelukast alkali salt obtained in step (e) is concentrated and converted into the amorphous montelukast alkali salts, as known in the art or by dissolving concentrated material into an organic solvent including but not limited to C1-C2 halogenated solvent may be selected from chloroform, dichloromethane or dichloroethane, preferably dichloromethane or C7-C8 aromatic hydrocarbon solvent may be selected from toluene, ethyl benzene or xylene, preferably toluene and isolating amorphous montelukast alkali salts by adding the obtained reaction mixture into the antisolvent including but not limited to C5-C7 acyclic solvent may be selected from pentane, hexane, n-hexane; n-heptane or n-octane, preferably hexane or n-heptane or C5-C8 cyclic hydrocarbon solvent may be selected from cyclopentane, cyclohexane or cycloheptane, preferably cyclohexane.

In another way, montelukast Benzhydryl piperazine salts represented by the formula (III) is combined with one or more solvent selected from the group consisting of water, water immiscible organic solvent such as aromatic hydrocarbon (benzene or toluene), aliphatic hydrocarbon (hexane or heptane), an aliphatic ester (ethyl acetate, etc.) or chlorinated hydrocarbon (chloroform, dichloromethane) at 20-40 °C. This suspension treated with a source of alkali metal, for instance alkali used in step (e) may be selected from sodium methoxide, sodium ethoxide, calcium methoxide, calcium ethoxide, potassium methoxide, potassium ethoxide, magnesium methoxide, magnesium ethoxide, etc. preferably the alkali is sodium methoxide, in suitable solvent system, preferably in an alcoholic solvent such as methanol, ethanol, propanol, butanol, 2-propanol or tertiary butanol. Then further work-up process remains same as mentioned above.

The pharmaceutical compositions of the present invention comprise Benzhydryl piperazine salts of Montelukast represented by the formula (III), prepared according to present invention, as an active ingredient and may also contain a pharmaceutically acceptable carrier, diluent, excipient, additive, filler, lubricant, solvent, binder, stabilizer and the like and optionally other ingredients used in pharmaceutical formulations. The compositions may also comprise one or more additional therapeutic agents. The compositions of this invention include compositions suitable for oral, rectal, topical, parenteral, ocular, pulmonary, or nasal administration, although the most suitable route in any given case will depend on the nature and severity of the conditions being treated and on the nature of the active ingredient. The compositions may be conveniently presented in unit dosage form and prepared by any of the methods well known in the art of the pharmacy.

Dosage forms include tablets, troches, dragees, powders, syrups, patches, liposomes, injections, dispersions, suspensions, solutions, capsules, creams, ointments and aerosols. Compositions which provide from 0.1 to 10.0 mg of the active ingredient are preferred.

In general, an effective amount means that amount of a compound of this invention that will elicit the biological or medical response that is being sought. Any suitable route of administration may be employed for providing a mammal, especially a human with an effective dosage of a compound of the present invention. For example, oral, rectal, topical, parenteral, ocular, pulmonary and nasal administration can be employed.

The processes described in the present invention were demonstrated in examples illustrated below. These examples are provided as illustration only and therefore should not be construed as limitation of the scope of the invention.

### Example-1:

### 2-(2-(3(S)-(3-(2-(7-Chloro-2-quinolinyl) ethenyl) phenyl)-3-methanesulfonyloxy propyl phenyl)-2-propanol

A 1.0 L round bottom flask fitted with a mechanical stirrer, thermocouple, and addition funnel was purged with nitrogen. The flask was charged with 2-(3(S)-(3-(2-(7-Chloro-2-quinolinyl)- ethenyl) phenyl)-3-hydroxypropyl)phenyl)-2-propanol (37 g) in toluene (96 ml) and reaction mixture was heated at 65-70°C to get clear solution, followed by addition with acetonitrile (242 ml). The solution and was cooled to -33 ± 3 °C and diisopropylethylamine (17.7 g) was added. Then methanesulfonyl chloride (9.7 gm diluted in 37 ml acetonitrile) was added dropwise over 25-30 minutes, keeping the temp. -33 ± 3°C. After the addition of methanesulfonyl chloride the reaction mixture was seeded with seed of the title compound (5 mg) to afford a thin slurry having solid compound was further added with acetonitrile (111 ml) and stirred at -33 ± 3 °C for 1 hour. The product was isolated by filtration of the cold suspension under a blanket of N2. The filter cake was washed with cold acetonitrile (111 ml), the cake was stored at <-10°C (wet wt. = 84 g).

### Example-2

### [R-(E)]-1-[[[1-[3-[2-(7-chloro-2-quinolinyl)ethenyl]phenyl]-3-[2-(1-hydroxy-1-methylethyl) Phenyl] Propyl] thio] methyl] cyclopropaneacetic acid (II)

A 1.0 L round bottom flask fitted with a mechanical stirrer, thermocouple, and addition funnel was purged with nitrogen. The flask was charged with 1-(mercaptomethyl)cyclopropaneacetic acid methyl ester (12.9 g) and dimethylformamide (150 ml) and reaction mixture was cooled to -2±2 °C. Added 2-(2-(3(S)-(3-(2-(7-Chloro-2-quinolinyl) ethenyl) phenyl)-3-methanesulfonyloxypropyl phenyl)-2-propanol in DMF to the reaction mixture at -2 ± 2 °C and then potassium-t-butoxide (9 g) was added. Stirred the reaction mixture for 2 hrs. and 890 ml water was added to the reaction mixture. The reaction mixture was extracted with ethyl acetate (370 ml) and concentrated the ethyl acetate layer completely under vacuum at 43-47°C, followed by adding isopropyl alcohol to the residue at 27-33°C. Added sodium hydroxide solution (9.7 g in 90 ml water) and stirred at a temperature of about 27-33°C for 3 hours and added with water (576 ml). Extracted the reaction mixture with the mixture of toluene (135 ml) and heptane (135 ml) and removed the organic layer. Acidify an aqueous layer with acetic acid solution and extracted the reaction mixture with ethyl acetate (370 ml), washed the organic layer with 1% NaHCO3 solution (370ml). Added acetic acid (0.8 ml) in ethyl acetate layer and ethyl acetate layer was washed with 1% NaHCO3 solution (180ml) (repeat twice) and ethyl acetate layer was distilled out under vacuum to get title compound in residual form (weight = 29.7 g).

### Example-3

### Crude Montelukast Benzhydryl piperazine Salt (III-a)

29.7 gm (~ 80% purity)of Montelukast acid (obtained from example-2) was dissolved in ethyl acetate (300 ml). 12.8 g of Benzhydryl piperazine was added at once and mixture was stirred for 15 min. Heptane (150 ml) was added slowly to the reaction mass, resulting dense mass was stirred for 3 hrs. More 150 ml Heptane was added slowly and stirred for 3 hrs. After filtering, washed with mixture of 60 ml of ethyl acetate and 60 ml of heptane and dried for 6 hrs. under vacuum at 40-48° C to get crude Montelukast Benzhydryl piperazine salt. (Weight = 32.3 g & Purity = 98.56%)

### Example-4

### Crude Montelukast Benzhydryl piperazine Salt (III-a)

In 11.2 gm('70% purity) of Montelukast acid in ethyl acetate (114 ml), 3.85 g of Benzhydryl piperazine was added at once and mixture was stirred for 15 min. Heptane (57 ml) was added slowly to the reaction mass, resulting dense mass was stirred for 3 hrs. More 57 ml Heptane was added slowly and stirred for 3 hrs. After filtering, washed with mixture of 15 ml of ethyl acetate and 15 ml of heptane and dried for 6 hrs. under vacuum at 40-48° C to get crude Montelukast Benzhydryl piperazine salt. (Weight = 12.18 g & Purity = 98%)

### Example-5

### Crude Montelukast 4-chloro-benzhydryl piperazine Salt (III-b)

21.0 gm of Montelukast acid (obtained according to example-2) was dissolved in isopropyl alcohol (210 ml). 10.3 g of 4-chloro-benzhydryl piperazine was added at once and mixture was stirred for 15 min., distilled out the isopropyl alcohol under vacuum. Obtained residue was dissolved in ethyl acetate (210 ml) and mixture was cooled to 27-30 °C. Heptane (150 ml) was added slowly to the reaction mass, resulting dense mass was stirred for 3 hrs. More 150 ml Heptane was added slowly and stirred for 7 hrs. After filtering, washed with mixture of 30 ml of ethyl acetate and 30 ml of heptane and dried for 6 hrs. under vacuum at 40-48° C to get crude Monielukast 4-chloro-benzhydryl piperazine salt. (Weight = 21 g & Purity = 98.97%)

### Example-6

### Pure Montelukast Benzhydryl piperazine Salt (III-a)

31 gm of Montelukast Benzhydryl piperazine salt and 93 ml. of methanol were charged in RBF under N2 atmosphere and mixture was heated to 55-60°C to get the clear solution. Solvent was distilled out under vacuum and residue was dissolved in ethyl acetate (310 ml) at 54-60° C, reaction mass was cooled to 27-33°C. 310 ml heptane was added slowly to the reaction mass, resulting mass was stirred for 3 hrs. After filtering, washed with mixture of 60 ml of ethyl acetate and 60 ml of heptane and dried for 6 hrs. under vacuum at 40-48° C to get pure Montelukast Benzhydryl piperazine salt (weight = 28 g & purity = 99.57%).

### Example-7

### Pure Montelukast 4-chloro-benzhydryl piperazine Salt (III-b)

13 g of Montelukast 4-chloro-benzhydryl piperazine salt and 100 ml. of ethyl acetate were charged in RBF under nitrogen atmosphere, 160 ml heptane was added slowly to the reaction mass, resulting mass was stirred for 3 hrs at 27-33°C. After filtering, washed with mixture of 20 ml of ethyl acetate and 40 ml of heptane and dried for 6 hrs. under vacuum at 40-48° C to get pure Montelukast 4-chloro-benzhydryl piperazine salt (weight = 11.3 g & purity = 99.44%).

### Example-8

### Amorphous Montelukast sodium from Montelukast Benzhydryl piperazine salt

Montelukast Benzhydryl piperazine salt (12 g), water (120 ml) and ethyl acetate (60 ml) were charged in RBF under nitrogen atmosphere at 27-33° C, followed by addition of acetic acid (3.4 g). The mixture was stirred for 15 min. at 27-33° C, followed by further addition of ethyl acetate (60 ml) and stirred for 15 min. at 27-33° C. The layers were separated and ethyl acetate layer was washed with water (2x60 ml) and organic layer thus obtained was dried over sodium sulfate. The reaction solution was decanted; solvent from the reaction mass was distilled out under vacuum to afford the residual mass. The obtained residual mass was combined with methanolic sodium hydroxide (0.6 gm NaOH in 36 ml methanol) and stirred for 15 minute at 44-50°C, followed by distillation of solvent from the reaction mass under vacuum at 44-50°C. The obtained residual mass was dissolved in toluene (72 ml) and added with 0.6 gm activated charcoal, filtered through hyflow bed and washed with hot toluene (24 ml), followed by distillation under vacuum at 54-60°C. The residue thus obtained was further dissolved in toluene (36 ml) and was slowly added to heptane (480 ml) under nitrogen atmosphere and stirred for 15 minutes at 27-33° C. The desired compound was filtered under nitrogen atmosphere and washed with heptane (40 ml). The wet compound was dried under vacuum at 40-46° C for 24 hrs to yield amorphous montelukast sodium (dry weight = 7.2 g & purity = 99.44%).

### Example-9

### Amorphous Montelukast sodium from Montelukast 4-chloro-benzhydryl piperazine salt

Montelukast 4-chloro-benzhydryl piperazine salt (11 g) and ethyl acetate (110 ml) were charged in RBF under nitrogen atmosphere at 27-33° C, followed by addition of acetic acid (3.0 gm). The mixture was stirred for 15 min. at 27-33° C, followed by further addition of water (110 ml) & ethyl acetate (55 ml) and stirred for 15 min. at 27-33° C. The layers were separated and ethyl acetate layer was washed with water (2×110 ml) and organic layer thus obtained was dried over sodium sulfate. The reaction solution was decanted; solvent from the reaction mass was distilled out under vacuum to afford the residual mass. The obtained residual mass was combined with methanolic sodium hydroxide (0.5 g NaOH in 33 ml methanol) and stirred for 15 minute at 42-48°C, followed by distillation of solvent from the reaction mass under vacuum at 42-48°C. The obtained residual mass was dissolved in toluene (66 ml) added with 0.6 g activated charcoal, filtered through hyflow bed and washed with hot toluene (24 ml), followed by distillation under vacuum at 54-60°C. The residue thus obtained was further dissolved in toluene (33 ml) and was slowly added to heptane (440 ml) under nitrogen atmosphere and stirred for 15 minutes at 27-33° C. The desired compound was filtered under nitrogen atmosphere and washed with heptane (44 ml). The wet compound was dried under vacuum at 40-46° C for 24 hrs to yield amorphous montelukast sodium (dry weight = 5.6 g & purity = 99.34%).

## Claims

1. A Benzhydryl piperazine salt of Montelukast represented by the formula (III); 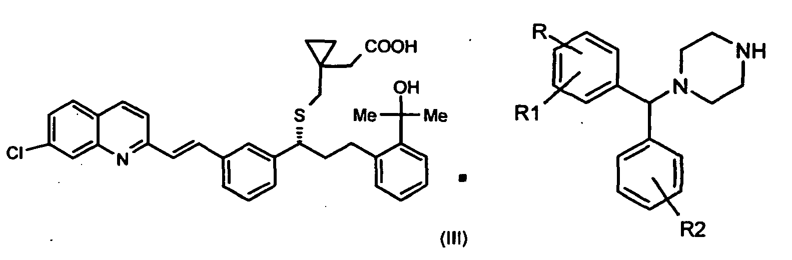 wherein;
R, R1, R2 is independently selected from hydrogen, halogen like F, Cl, Br & I, C1-C6 alkyl, (C1-C6) alkoxy, CH2F, CHF2 and CF3.

2. The Benzhydryl piperazine salt of Montelukast according to claim 1, wherein R is H, Cl, methoxy, F, methyl or CF3; R1 is H or Cl and R2 is H.

3. The Benzhydryl piperazine salt of Montelukast according to claim 1, which is selected from the group, consisting of:
(a) Montelukast Benzhydryl Piperazine (III-a), wherein R = H, R1= H, R2 = H.
(b) Montelukast 4-Chloro-benzhydryl piperazine (III-b), wherein R= 4-Cl, R1 = H, R2 = H.
(c) Montelukast 4-methoxy-benzhydryl piperazine (III-c), wherein R=4-OMe, R1= H, R2=H.
(d) Montelukast 3,5-dichloro-benzhydryl piperazine (III-d), wherein R=3-Cl, R1=5-Cl, R2=H.
(e) Montelukast 3,4-dichloro-benzhydryl piperazine (III-e), wherein R=3-Cl, R1=4-Cl, R2=H
(f) Montelukast 4-fluoro-benzhydryl piperazine (III-f), wherein R=4-F, R1=H, R2=H
(g) Montelukast 4-methyl-benzhydryl piperazine (III-g), wherein. R=4-Me, R1=H, R2=H and
(h) Montelukast 4-trifluoromethyl-benzhydryl-piperazine (III-h), wherein R=4-CF3, R1=H, R2=H

4. The use of Benzhydryl piperazine salt of Montelukast as claimed in claim 1 in the preparation of Montelukast or its alkali salts.

5. A process for preparing the Montelukast benzhydryl piperazine salt represented by the formula (III), which comprises:
(a) forming a solution of Montelukast of formula (II) in suitable solvent,
(b) adding benzhydryl piperazine derivative of formula (IV) to said solution obtained in step (a), 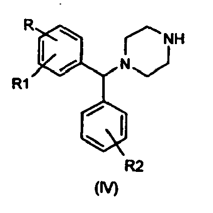 wherein;
R, R1, R2 is independently selected from hydrogen, halogen like F, Cl, Br & I, (C1-C6) alkyl, (C1-C6) alkoxy, CH2F, CHF2 and CF3.
(c) isolating Benzhydryl piperazine salt of montelukast represented by the formula (III),
(d) optionally recrystallizing the benzhydryl piperazine salt of Montelukast represented by the formula (III) obtained in step (c),

6. A process for preparing the Montelukast or its alkali salts, which comprises:
(a) forming a solution of Montelukast of formula (II) in suitable solvent,
(b) adding benzhydryl piperazine derivative of formula (IV) to said solution obtained in step (a), 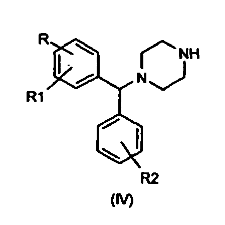 wherein;
R, R1, R2 is independently selected from hydrogen, halogen like F, Cl, Br & I, (C1-C6) alkyl, (C1-C6) alkoxy, CH2F, CHF2 and CF3.
(c) isolating Benzhydryl piperazine salt of montelukast represented by the formula (III),
(d) optionally recrystallizing the benzhydryl piperazine salt of Montelukast represented by the formula (III) obtained in step (c),
(e) converting the said benzhydryl piperazine salt of Montelukast represented by the formula (III) into substantially pure Montelukast or its alkali salts.

7. The process according to claim 5 or 6, wherein solvent used is selected from the group comprising of alcohols, ketones, aliphatic ethers, cyclic ethers, aliphatic esters, hydrocarbons, chlorinated solvent, acetonitrile and mixtures thereof.

8. The process according to claim 6, wherein said conversion of said benzhydryl piperazine salt of Montelukast comprises forming a solution of said benzhydryl piperazine salt of Montelukast in a mixture of organic solvent and water; treating the mixture with acid; and extracting montelukast from the organic phase; optionally treating with alkali metal.

9. The process according to claim 8, wherein alkali metal is sodium hydroxide.

10. A pharmaceutical composition comprising the Benzhydryl piperazine salt of Montelukast as claimed in claim and at least one pharmaceutically acceptable carriers of excipients.

## Patentansprüche

1. Benzhydrylpiperazin-Salz von Montelukast, wie dargestellt durch die Formel (III): 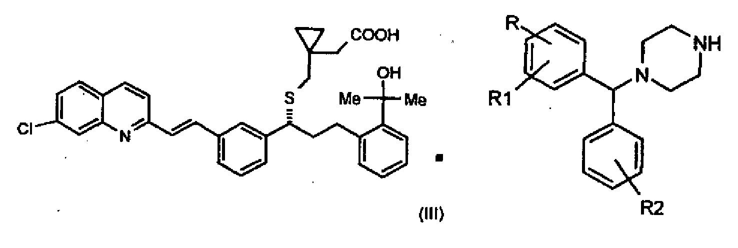 worin:
R, R1, R2 unabhängig ausgewählt sind aus Wasserstoff, einem Halogen wie F, Cl, Br und I, (C1-C6)-Alkyl, (C1-C6)-Alkoxy, CH2F, CHF2 und CF3.

2. Benzhydrylpiperazin-Salz von Montelukast nach Anspruch 1, worin R H, Cl, Methoxy, F, Methyl oder CF3 ist; R1 H oder Cl ist und R2 H ist.

3. Benzhydrylpiperazin-Salz von Montelukast nach Anspruch 1, welches ausgewählt ist aus der Gruppe, bestehend aus:
(a) Montelukast-Benzhydrylpiperazin (III-a), worin R = H, R1 = H, R2 = H.
(b) Montelukast-4-chlor-benzhydrylpiperazin (III-b), worin R = 4-Cl, R1 = H, R2=H.
(c) Montelukast-4-methoxy-benzhydrylpiperazin (III-c), worin R = 4-OMe, R1 = H, R2 = H.
(d) Montelukast-3,5-dichlor-benzhydrylpiperazin (III-d), worin R = 3-Cl, R1 = 5-Cl, R2 = H.
(e) Montelukast-3,4-dichlor-benzhydrylpiperazin (III-e), worin R = 3-Cl, R1 = 4-Cl, R2 = H.
(f) Montelukast-4-fluor-benzhydrylpiperazin (III-f), worin R = 4-F, R1 = H, R2=H.
(g) Montelukast-4-methyl-benzhydrylpiperazin (III-g), worin R = 4-Me, R1 = H, R2=Hund
(h) Montelukast-4-trifluormethyl-benzhydrylpiperazin (III-h), worin R = 4-CF3, R1 = H, R2 = H.

4. Vervvendung des Benzhydrylpiperazin-Salzes von Montelukast nach Anspruch 1 in der Herstellung von Montelukast oder seinen Alkalisalzen.

5. Verfahren zur Herstellung von Montelukast-Benzhydrylpiperazin-Salz, wie dargestellt durch die Formel (III), welches umfasst:
(a) Bilden einer Lösung von Montelukast der Formel (II) in einem geeigneten Lösungsmittel,
(b) Zusetzen des Benzhydrylpiperazin-Derivats der Formel (IV) zu der in Schritt (a) erhaltenen Lösung, 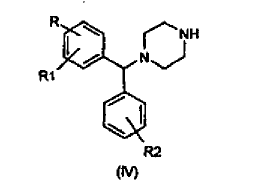 worin:
R, R1, R2 unabhängig ausgewählt sind aus Wasserstoff, einem Halogen wie F, Cl, Br und I, (C1-C6)-Alkyl, (C1-C6)-Alkoxy, CH2F, CHF2 und CF3,
(c) Isolieren des Benzhydrylpiperazin-Salzes von Montelukast, wie dargestellt durch die Formel (III),
(d) wahlweise Rekristallisieren des in Schritt (c) erhaltenen Benzhydrylpiperazin-Salzes von Montelukast, wie dargestellt durch die Formel (III).

6. Verfahren zur Herstellung von Montelukast oder seinen Alkalisalzen, welches umfasst:
(a) Bilden einer Lösung von Montelukast der Formel (II) in einem geeigneten Lösungsmittel,
(b) Zusetzen des Benzhydrylpiperazin-Derivats der Formel (IV) zu der in Schritt (a) erhaltenen Lösung, 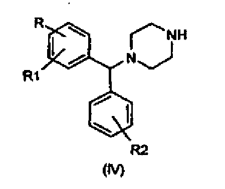 worin:
R, R1, R2 unabhängig ausgewählt sind aus Wasserstoff, einem Halogen wie F, Cl, Br und I, (C1-C6)-Alkyl, (C1-C6)-Alkoxy, CH2F, CHF2 und CF3,
(c) Isolieren des Benzhydrylpiperazin-Salzes von Montelukast, wie dargestellt durch die Formel (III),
(d) wahlweise Rekristallisieren des in Schritt (c) erhaltenen Benzhydrylpiperazin-Salzes von Montelukast, wie dargestellt durch die Formel (III),
(e) Umwandeln des Benzhydrylpiperazin-Salzes von Montelukast, wie dargestellt durch die Formel (III), zu im wesentlichen reinem Montelukast oder seinen Alkalisalzen.

7. Verfahren nach Anspruch 5 oder 6, wobei das verwendete Lösungsmittel ausgewählt ist aus der Gruppe, bestehend aus Alkoholen, Ketonen, aliphatischen Ethern, zyklischen Ethern, aliphatischen Estern, Kohlenwasserstoffen, chloriertem Lösungsmittel, Acetonitril und Gemischen davon.

8. Verfahren nach Anspruch 6, wobei die Umwandlung des Benzhydrylpiperazin-Salzes von Montelukast das Bilden einer Lösung des Benzhydrylpiperazin-Salzes von Montelukast in einem Gemisch aus organischem Lösungsmittel und Wasser; Behandeln des Gemischs mit Säure; und Extrahieren von Montelukast aus der organischen Phase; wahlweise Behandeln mit Alkalimetall, umfasst.

9. Verfahren nach Anspruch 8, wobei das Alkalimetall Natriumhydroxid ist.

10. Pharmazeutische Zusammensetzung, umfassend das Benzhydrylpiperazin-Salz von Montelukast nach Anspruch 1 und wenigstens einen pharmazeutisch akzeptablen Träger oder Exzipienten.

## Revendications

1. Sel de pipérazine de benzhydryle de Montélukast représenté par la formule (III) ; 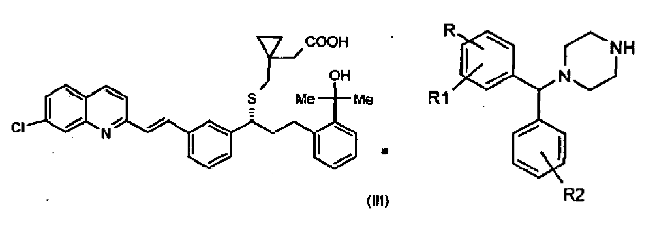 dans laquelle ;
R, R1, R2 sont indépendamment choisis parmi un hydrogène, un halogène tel que F, Cl, Br & I, un alkyle en C1-C6, un alcoxy en C1-C6, CH2F, CHF2 et CF3.

2. Sel de pipérazine de benzhydryle de Montélukast selon la revendication 1, dans lequel R est H, Cl, méthoxy, F, méthyle ou CF3 ; R1 est H ou Cl et R2 est H.

3. Sel de pipérazine de benzhydryle de Montélukast selon la revendication 1, qui est choisi dans le groupe constitué par :
(a) la pipérazine de benzhydryle de Montélukast (III-a), dans laquelle R=H, R1=H, R2=H,
(b) la pipérazine de 4-chloro-benzhydryle de Montélukast (III-b), dans laquelle R=4-C1, R1=H, R2=H,
(c) la pipérazine de 4-méthoxy-benzhydryle de Montélukast (III-c), dans laquelle R=4-OMe, R1=H, R2=H.
(d) la pipérazine de 3,5-dichloro-benzhydryle de Montélukast (III-d), dans laquelle R=3-C1, R1=5-C1, R2=H,
(e) la pipérazine de 3,4-dichloro-benzhydryle de Montélukast (III-e), dans laquelle R=3-C1, R1=4-C1, R2=H,
(f) la pipérazine de 4-fluoro-benzhydryle de Montélukast (III-f), dans laquelle R=4-F, R1=H, R2=H,
(g) la pipérazine de 4-méthyl-benzhydryle de Montélukast (III-g), dans laquelle R=4-Me, R1=H, R2=H et
(h) la pipérazine de 4-trifluorométhyl-benzhydryle de Montélukast (III-h), dans laquelle R=4-CF3, R1=H, R2=H.

4. Utilisation du sel de pipérazine de benzhydryle de Montélukast selon la revendication 1, dans la préparation de Montélukast ou de ses sels alcalins.

5. Procédé de préparation du sel de pipérazine de benzhydryle de Montélukast représenté par la formule (III), qui comprend :
(a) la formation d'une solution de Montélukast de formule (II) dans un solvant adapté,
(b) l'ajout d'un dérivé de pipérazine de benzhydryle de formule (IV) à ladite solution obtenue dans l'étape (a), 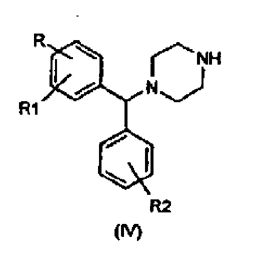 dans laquelle ;
R, R1, R2 sont indépendamment choisis parmi un hydrogène, un halogène tel que F, Cl, Br & I, un alkyle en C1-C6, un alcoxy un C1-C6, CH2F, CHF2 et CF3,
(c) l'isolement du sel de pipérazine de benzhydryle de Montélukast représenté par la formule (III),
(d) la recristallisation facultative du sel de pipérazine de benzhydryle de Montélukast représenté par la formule (III) obtenu dans l'étape (c).

6. Procédé de préparation du Montélukast ou de ses sels alcalins, qui comprend :
(a) la formation d'une solution de Montélukast de formule (II) dans un solvant adapté,
(b) l'ajout d'un dérivé de pipérazine de benzhydryle de formule (IV) à ladite solution obtenue dans l'étape (a), 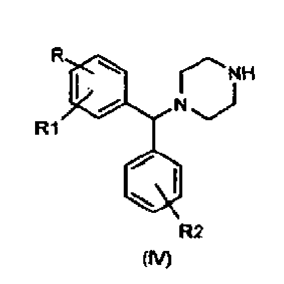 dans laquelle ;
R, R1, R2 sont indépendamment choisis parmi un hydrogène, un halogène tel que F, Cl, Br & I, un alkyle en C1-C6, un alcoxy un C1-C6, CH2F, CHF2 et CF3,
(c) l'isolement du sel de pipérazine de benzhydryle de Montélukast représenté par la formule (III),
(d) la recristallisation facultative du sel de pipérazine de benzhydryle de Montélukast représenté par la formule (III) obtenu dans l'étape (c),
(e) la conversion dudit sel de pipérazine de benzhydryle de Montélukast représenté par la formule (III) en le Montélukast sensiblement pur ou en ses sels alcalins.

7. Procédé selon la revendication 5 ou 6, dans lequel le solvant utilisé est choisi dans le groupe comprenant les alcools, les cétones, les éthers aliphatiques, les éthers cycliques, les esters aliphatiques, les hydrocarbures, un solvant chloré, l'acétonitrile et leurs mélanges.

8. Procédé selon la revendication 6, dans lequel ladite conversion dudit sel de pipérazine de benzhydryle de Montélukast comprend la formation d'une solution dudit sel de pipérazine de benzhydryle de Montélukast dans un mélange de solvant organique et d'eau ; le traitement du mélange à l'acide ; et l'extraction de montélukast de la phase organique ; le traitement facultatif avec un métal alcalin.

9. Procédé selon la revendication 8, dans lequel le métal alcalin est l'hydroxyde de sodium.

10. Composition pharmaceutique comprenant le sel de pipérazine de benzhydryle de Montélukast selon la revendication 1 et au moins un vecteur ou un excipient pharmaceutiquement acceptables.
